# EUROPÄISCHE PATENTSCHRIFT

(11) **EP 0 647 628 B1**
(45) Veröffentlichungstag und Bekanntmachung des Hinweises auf die Patenterteilung: **11.09.2002**
(21) Anmeldenummer: 94119395.5
(22) Anmeldetag: 19.08.1987
(51) Int. Cl.: C07D 219/04, G01N 33/533, C07D 401/12, G01N 33/68

(54) **Acridiniumderivate und ihre Verwendung in Lumineszenzimmunoassays**
Acridinium derivatives and their use in luminescence immunoassays
Dérivés de l'acridinium et leur utilisation dans des tests d'immunoluminescence

(30) Priorität: 22.08.1986 DE 3628573
(43) Veröffentlichungstag der Anmeldung: 12.04.1995
(62) Teilanmeldung aus: 87112022.6
(73) Patentinhaber: Dade Behring Marburg GmbH, 35001 Marburg (DE)
(72) Erfinder: Molz, Peter, Dr., D-55118 Mainz (DE); Skrzipczyk, Heinz-Jürgen, Dr., D-63263 Zeppelinheim (DE); Lübbers, Henning, Dr., D-65825 Schwalbach am Taunus (DE); Strecker, Helmut, Dr., D-64319 Pfungstadt (DE); Schnorr, Gerd, Dr., D-61118 Bad Vilbel (DE); Kinkel, Tonio, Dr., D-65830 Kriftel (DE)

(56) Entgegenhaltungen:
- EP-A- 0 082 636
- GB-A- 1 461 877
- GB-A- 2 008 247
- CLINICAL CHEMISTRY, Bd.28, Nr.8, August 1983 Seiten 1474 - 1479 I. WEEKS ET AL. 'Acridinium esters as high-specific-activity labels in immunoassay'

## Beschreibung

Der Gegenstand der Erfindung sind chemilumineszierende Acridinderivate, Verfahren zu ihrer Herstellung und ihre Verwendung in Lumineszenzimmunoassays.

Lumineszierende Verbindungen finden bereits vielseitige Anwendung. Sie werden als Indikatoren in Bioassays, Enzymimmunoassays und Lumineszenzimmunoassays eingesetzt (vgl. W.P. Collins "Alternative Immunoassays", Verlag John Wiley & Sons Ltd., Chichester, 1985), werden aber auch in Nukleinsäure-Hybridisierungsassays verwendet (vgl. J.A. Matthews et al. "Analytical Biochemistry", 151, 205-209, 1985). Außerdem werden chemilumineszierende Verbindungen bei der "flow injection analysis", in post-column-Detektoren in der Flüssigkeitschromatographie, in der Strömungsforschung und in künstlichen Lichtquellen eingesetzt.

Bei den Chemilumineszenzimmunoassays haben insbesondere zwei Strukturtypen von chemilumineszierenden Markierungssubstanzen größere Bedeutung erlangt. Es handelt sich hierbei einerseits um die Luminol- bzw. Isoluminolderivate, die bei H.R. Schroeder et al., "Methods in Enzymology", Academic Press Inc., New York, Vol. LVII, 1978, 424 ff sowie in den britischen Patenten 2 008 247 und 2 041 920, den deutschen Patentschriften 26 18 419 und 26 18 511, sowie in der europäischen Patentanmeldung 135 071 beschrieben sind. Einen Überblick über die praktische Anwendung der Isoluminolverbindungen als Lumineszenzindikatoren findet man bei W.G. Wood, J. Clin.Chem. Clin. Biochem. 22, 1984 905-918.

Andererseits haben auch Acridiniumesterverbindungen als chemilumineszierende Markierungssubstanzen Anwendung gefunden. Derartige Acridiniumester sind aus dem US-Patent 3 352 791, den britischen Patenten 1 316 363 und 1 461 877 sowie aus der europäischen Patentanmeldung 82 636 bekannt. Die Anwendung der Acridiniumester als Markierungssubstanzen in Immunoassays ist bei Weeks et al., Clin. Chem. 29/8 (1983), 1474-1479, beschrieben. Auch die Verwendung von Phenanthridiniumestern als Markierungssubstanz in Lumineszenzimmunoassays ist aus der europäischen Patentanmeldung 170 415 bereits bekannt.

Die Chemilumineszenz von Acridiniumestern kann durch Zugabe von alkalischer H₂O₂-Lösung gestartet werden. Für den Mechanismus der Chemilumineszenz hat F. McCapra, Acc.Chem. Res. 9, 201, 1976, eine überzeugende Erklärung gegeben. Dabei ist offensichtlich die Art der Abgangsgruppe sowohl für die Lichtquantenausbeute als auch für die hydrolytische Stabilität entscheidend.

Die bisher schon bekannten Acridiniumester haben gegenüber den Luminol- und Isoluminolverbindungen den Vorteil einer höheren Lichtquantenausbeute, die auch durch an den Indikator gebundene Proteine nicht beeinträchtigt wird (vgl. Weeks et al., Clin. Chem. 29/8 (1983), 1474-1479).

Obwohl die aus der europäischen Patentanmeldung 82 636 bekannten Acridiniumphenylester bei Anregung der Chemilumineszenz durch milde Oxidationsmittel sich durch eine hohe Nachweisempfindlichkeit auszeichnen, weisen sie für die praktische Anwendung störende Nachteile auf. Vor allem ist die Phenylesterbindung in wäßrigen Systemen auch schon bei Raumtemperatur sehr labil. Hinzu kommt, daß unter den dort angegebenen Oxidationsbedingungen die Acridiniumphenylester eine Lichtemission zeigen, die erst nach etwa 10 Sekunden weitgehend, d.h. zu über 95 %, abgeklungen ist. Im Vergleich dazu haben andere nicht isotopische Assayverfahren weitaus kürzere Meßzeiten und ermöglichen dadurch einen höheren Probendurchsatz.

Aufgabe der vorliegenden Erfindung war es deshalb, neue Acridiniumderivate zur Verfügung zu stellen, die bei hoher Lichtquantenausbeute eine schnellere Reaktionskinetik aufweisen und damit kurze Meßzeiten für einen Lumineszenzimmunoassay ermöglichen.

Es wurde nun gefunden, dass diese Anforderungen erfüllt werden von lumineszenten Verbindungen, gekennzeichnet durch ein Acridiniumderivat der Formel I 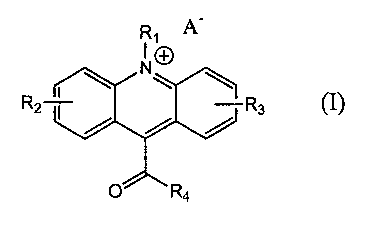 in der R₁ Wasserstoff, ein Alkyl-, Alkenyl- oder Alkinylrest mit 1 bis 10 Kohlenstoffatomen, eine Benzyl-, Phenyl- oder Naphtylgruppe ist, R₂ und R₃ Wasserstoff, eine Alkylgruppe mit 1 bis 4 Kohlenstoffatomen, eine Aminogruppe oder eine mono- oder di-C₁-C₄ Alkylaminogruppe ist, wobei die Alkylreste mit Hydroxy monosubstituiert sein können, sowie Morpholin, eine Carboxy-, C₁-C₄-Alkoxy-, Cyano-, Nitrogruppe oder Halogen sind, R₄ einen Rest darstellt, in dem eine substituierte Sulfonamidgruppe über den Stickstoff direkt an die Carbonylgruppe gebunden ist und A⁻ ein die Chemilumineszenz nicht beeinträchtigendes Anion ist, ausgenommen sind Acridiniumverbindungen der Formel I, in denen R₁, R₂ und R₃ wie oben definiert sind und R₄ eine substituierte Sulfonamidgruppe der Formel V oder VI 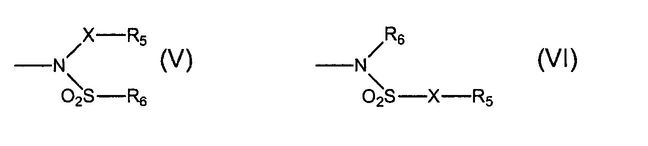 ist, wobei X eine verzweigte oder unverzweigte C₁-C₅-Alkylengruppe oder eine Phenylen- oder Naphthylengruppe ist oder Chinol, Indol, Pyrrol oder Pyridin ist, R₅ eine reaktive Gruppe ist, die unter schonenden Bedingungen selektiv mit Amino-, Carboxy-, Thiol- oder anderen funktionellen Gruppen in Substanzen von biologischem Interesse eine Bindung eingehen kann, und R₆ in der Formel V ein Alkyl- oder Alkenylrest mit 1 bis 10 Kohlenstoffatomen, eine mit C₁-C₄ Alkyl mono- oder disubstituierte Aminogruppe, eine Morpholino-, eine Benzyl- oder eine Phenyl- oder Naphthylgruppe ist, die ggf. mit Hydroxy, Amino, Alkoxy mit 1 bis 4 Kohlenstoffatomen oder Phenoxyoder Naphthoxygruppen substituiert ist, und R₆ in der Formel VI zusätzlich zu den genannten Substituenten auch noch Wasserstoff bedeuten kann, wobei das Acridiniumderivat der Formel I direkt oder über ein Brückenmolekül an ein Protein, ein Polypeptid oder an eine andere Substanz von biologischem Interesse unter Ausbildung eines stabilen, immunologisch aktiven Konjugates gebunden ist.

Unter den Substanzen von biologischem Interesse sind vor allem Antigene zu verstehen. Unter diesen Begriff fallen auch Hormone, Steroide, Arzneimittel, Arzneimittelmetabolite Toxine, Alkaloide und auch Antikörper.

Das die Chemilumineszenz nicht beeinträchtigende Anion kann ein Tetrafluoroborat-, Perchlorat-, Halogenid-, Alkylsulfat-, Halosulfonat-, Alkylsulfonat- oder Arylsulfonat-Anion sein. Auch jedes andere Anion kann eingesetzt werden, sofern es die Chemilumineszenz nicht löscht oder abschwächt.

Unter Aryl werden aromatische Kohlenwasserstoffe verstanden, insbesondere Phenyl und Naphthyl. Unter Heteroatome enthaltenden aromatischen Gruppen werden aromatische Kohlenwasserstoffe verstanden, die ein Stickstoff- oder Sauerstoffatom enthalten, insbesondere Chinolin, Indol, Pyrrol und Pyridin.
Unter Halogenen werden Fluor, Chlor, Brom und Jod verstanden.

Die substituierten Aminogruppen sind bevorzugt C₁-C₄-Alkyloder C₁-C₄-Dialkylamine, wobei die Alkylreste ihrerseits beispielsweise mit Hydroxy monosubstituiert sein können. Ebenso kann es sich bei den substituierten Aminen um einen Morpholinrest handeln. Die für R² und R³ angegebenen Alkoxyreste sind bevorzugt solche mit 1-4 C-Atomen im Alkylteil.

Die für X angegebenen verzweigten oder unverzweigten aliphatischen Gruppen sind bevorzugt C₁-C₅-Alkylengruppen.

Im allgemeinen werden Acridiniumderivate gewählt, in denen X eine Methylen-, Äthylen-, Propylen- oder eine ortho-, meta- oder para-Phenylengruppe ist. Zur Verbesserung der Wasserlöslichkeit der Acridiniumderivate können diese Gruppen auch Heteroatome enthaltende, hydrophile Substituenten tragen.

Von besonderer Bedeutung für die Einsatzmöglichkeiten der erfindungsgemäßen Acridiniumderivate ist der Substituent R⁵. Durch die geeignete Auswahl dieser Gruppe erhält das Acridinderivat eine so hohe Reaktivität, daß es schon unter milden Bedingungen selektiv mit einer funktionellen Gruppe der nachzuweisenden biologischen Substanz eine Bindung eingehen kann. Geeignete reaktive Gruppen sind aus der folgenden Zusammenstellung zu ersehen:

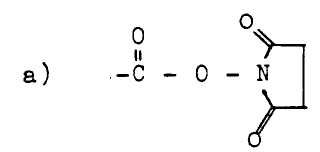

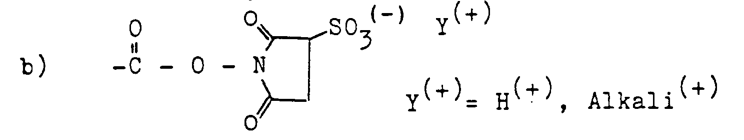

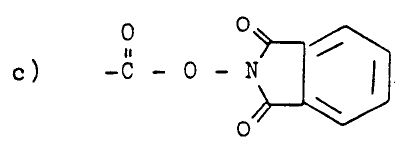

d) -SO₂ - CH = CH₂

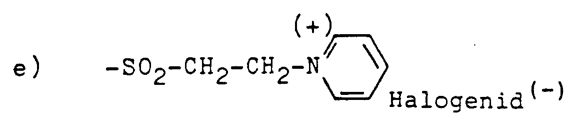

f) -N = C = S

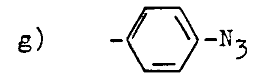

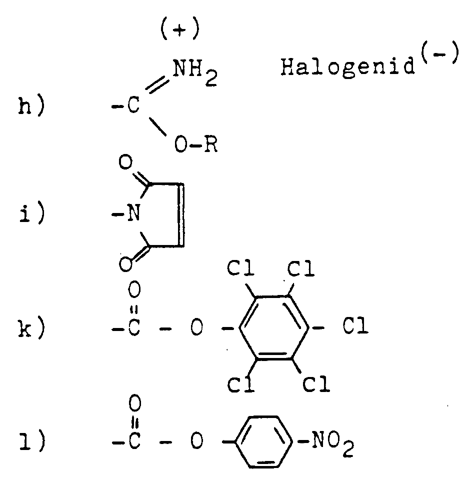

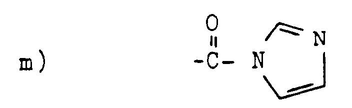

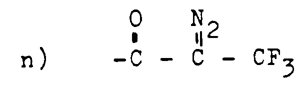

In vielen Fällen haben sich erfindungsgemäße Acridiniumderivate als geeignet erwiesen, bei denen R⁵ eine Gruppe der Formel III 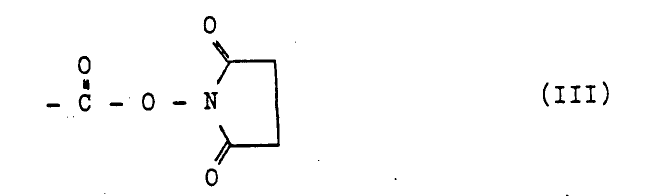 ist.

Außerdem werden auch Acridiniumverbindungen bevorzugt, in denen R¹ eine Methylgruppe ist. Besonders häufig werden deshalb erfindungsgemäße Acridiniumverbindungen eingesetzt, die der Formel IV 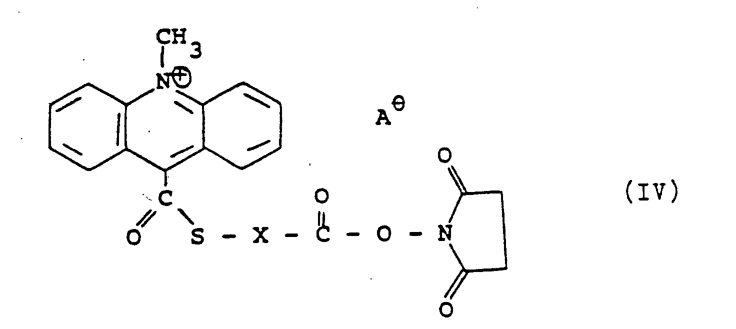 entsprechen, in denen A und X die vorstehend genannten Bedeutungen haben.

Wenn in den erfindungsgemäßen Acridiniumderivaten der Rest R⁴ ein Sulfonamid-Rest ist, dann kann er bevorzugt der Formel V 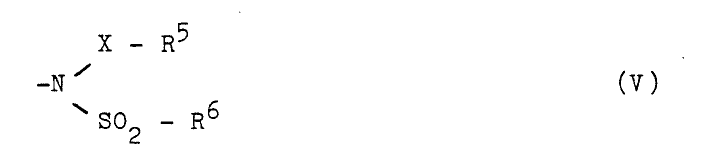 oder der Formel VI 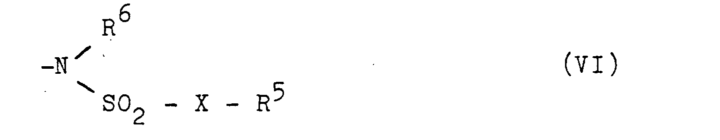 entsprechen, wobei X und R⁵ die obengenannten Bedeutungen haben und R⁶ in der Formel V ein Alkyl- oder Alkenylrest mit 1 bis 10 Kohlenstoffatomen, eine vorzugsweise mit C₁-C₄-Alkyl mono- oder disubstituierte Aminogruppe, eine Morpholino-, eine Benzyl- oder eine Arylgruppe ist, die auch mit Hydroxy, Amino, Alkoxy mit 1 bis 4 Kohlenstoffatomen oder Aryloxy substituiert sein können, und R⁶ in der Formel VI zusätzlich auch noch Wasserstoff bedeuten kann.

Typische Vertreter dieser erfindungsgemäßen Klasse von Acridiniumderivaten entsprechen der Formel VII 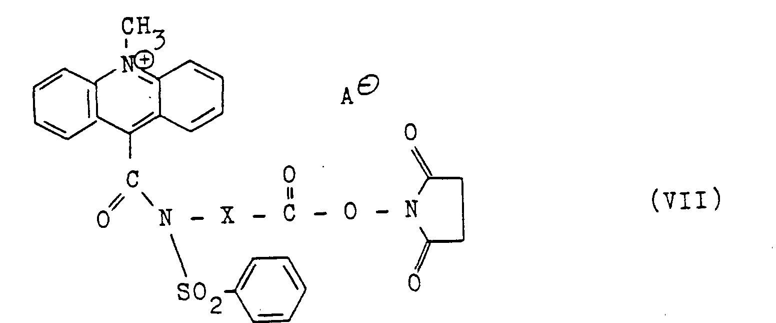 in der A und X die obengenannten Bedeutungen haben.

Durch die vorliegende Erfindung werden also zwei neue Klassen von Acridiniumverbindungen zur Verfügung gestellt, wobei die eine Klasse durch eine Thiolestergruppierung und die andere durch eine Sulfonamidstruktur sich auszeichnen. Die Acridiniumacylsulfonamid-Derivate weisen eine höhere Stabilität, die Thiolester eine schnellere Kinetik als die bisher bekannten Acridiniumphenylester-Verbindungen auf. Beide Verbindungsklassen zeichnen sich außerdem durch eine höhere Lichtausbeute aus.

Ein bedeutender Vorteil der erfindungsgemäßen Acridiniumverbindungen mit thiolesterhaltigen Abgangsgruppen gegenüber den aus der Europäischen Patentanmeldung 82 636 bekannten Acridiniumphenylestern liegt in der wesentlichen schnelleren Reaktionskinetik der Lichtemission. So liefert der nach Beispiel 1 hergestellte erfindungsgemäße Acridiniumthioester (Verbindung 6) bei 1 Sekunde Meßzeit eine um den Faktor 10 höhere Lichtausbeute unter den gleichen Oxidationsbedingungen. Diese hohe Lichtausbeute bei gleichzeitig kurzen Meßzeiten ermöglicht einen wesentlich höheren Probendurchsatz im Luminometer.

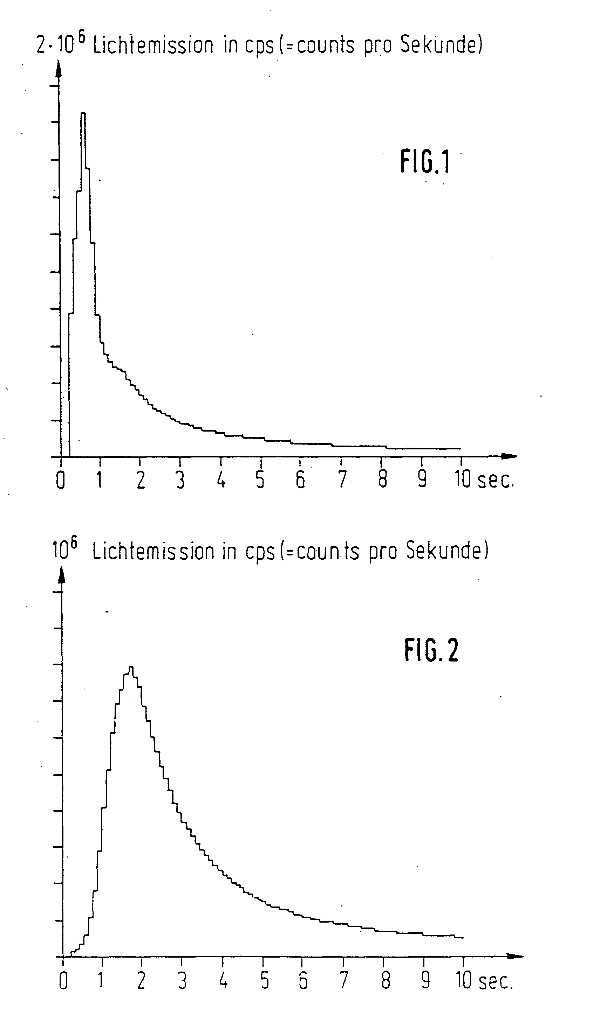

So zeigt Fig. 1 die Kinetik der Lichtemission eines Antikörperkonjugates des nach Beispiel 1 hergestellten Acridiniumthioesters (Verbindung 6)) und Fig. 2 die des Antikörperkonjugates des 4-(2-Succinimidyl-oxycarbonylethyl)-phenyl-10-methylacridinium-9-carboxylat-methosulfats (Europäische Patentanmeldung 82 636, Seite 10). 100 µl der jeweiligen Tracerlösungen werden durch Zugabe von 350 µl 0.05 m KCl/NaOH-Puffer pH 13 + 0.1 % H₂O₂ zur Chemilumineszenz angeregt und über einen Zeitraum von 10 Sekunden aufgezeichnet.

In Fig. 1 wird die maximale Lichtemission schon nach 0,66 Sekunden erreicht und ist nach 0,88 Sekunden schon wieder auf die Hälfte abgefallen. Demgegenüber wird in Fig. 2 die maximale Lichtemission erst nach 1,77 Sekunden erreicht und ist nach 2,88 Sekunden erst wieder auf die Hälfte abgefallen.

Völlig unerwartet ist, daß am Amidstickstoff sulfonylsubstituierte Acridinium-9-carbonsäureamide eine ausgezeichnete Chemilumineszenz aufweisen, denn es ist bekannt, daß Acridinium-9-carbonsäureamid im Gegensatz zu den Acridinium-9-carbonsäureestern keinerlei Chemilumineszenz zeigt (vgl. F. McCapra in W. Carruthers und J.K. Sutherland: Progress in Organic Chem., Vol. 8, 231-277, 1973, Butterworth, London).
Die erfindungsgemäßen Acridinium-9-carbonsäurethioester lassen sich auf folgendem Wege herstellen:
Acridin oder dessen Derivate mit R² und/oder R³ in den ankondensierten Phenylringen werden nach dem von Lehmstedt und Hundertmark in Ber. 63, 1229 (1930) angegebenen Verfahren in Ethanol/Eisessig und Kaliumcyanid zum 9-Cyanacridin umgesetzt. Hieraus wird nach Umkristallisation durch Umsetzung mit Schwefelsäure und Natriumnitrit entsprechend dem von Lehmstedt und Wirth in Ber. 61, 2044 (1928) beschriebenen Verfahren die Acridin-9-carbonsäure bzw. R²/R³ substituierte Acridin-9-carbonsäure gewonnen. Durch Umsetzung der Acridin-9-carbonsäure bzw. R²/R³ substituierten Acridin-9-carbonsäure mit Thionylchlorid wird die Verbindung der Formel VIII 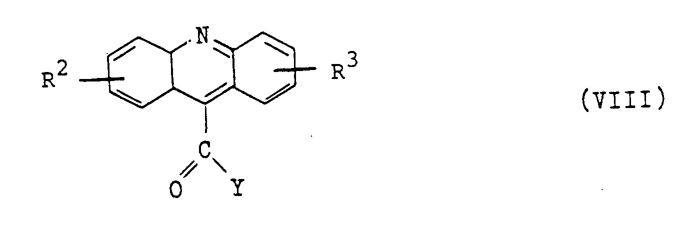 gewonnen, in der Y die Bedeutung von Chlor hat. Anstelle eines Halogens kann in die Verbindung VIII für Y auch eine Oxycarbonyl-C₁-C₅-alkyl-, Oxycarbonylaryl- oder Imidazolidgruppe eingeführt werden.

Die R²/R³-substituierten Acridinderivate können nach literaturbekannten Verfahren einfach synthetisiert werden. Solche Synthesen sind beispielsweise beschrieben in: Comprehensive Heterocyclic Chemistry; Editors A. Katritzky, C.W. Rees, Vol. 2, S. 395ff, Pergamon Press, 1984 oder Heterocyclic Compounds, Vol. 9, Acridines and Cond. 2^{nd} Edition, R.M. Acheson, John Wiley and Sons, 1973.

Das Säurechlorid (VIII) wird dann mit einer Thiolcarbonsäure der Formel IX,

HS - X - COOH (IX)

z.B. mit der 2-Mercaptobenzoesäure unter alkalischen Bedingungen zur Thiolestercarbonsäure umgesetzt, die anschließend mit einer zur Herstellung des Restes R⁵ geeigneten Verbindung, beispielsweise mit N-Hydroxysuccinimid, verestert wird. Anschließend wird die Acridinverbindung nach literaturbekannten Verfahren in 10-Stellung alkyliert. Für eine Methylierung eignet sich vor allem Trimethyloxoniumtetrafluoroborat, jedoch werden auch mit Dimethylsulfat, Methylfluorsulfonat, Toluolsulfonsäuremethylester oder Trifluoromethansulfonsäuremethylester gute Ausbeuten der chemilumineszierenden Acridiniumverbindung erhalten.

Zur Herstellung der erfindungsgemäßen Acridinium-Sulfonamidderivate wird ebenfalls vom Acridin-9-carbonsäurechlorid (VIII) ausgegangen. Diese Verbindung wird dann mit einem primären oder sekundären Sulfonamid, vorzugsweise mit einer geschützten Sulfonamidcarbonsäure der Formel X 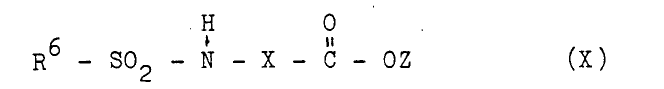 oder der Formel XI 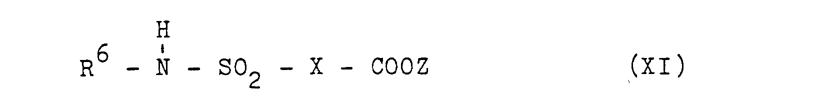 umgesetzt, in denen X und R⁶ die vorstehend genannten Bedeutungen haben und Z ein die Carboxygruppe schützender Rest ist, der anschließend abgespalten wird. Beispielsweise kann für diese Reaktion der N-Benzolsulfonylglycinbenzylester als Schutzgruppe eingesetzt werden. Die nach Abspaltung der Schutzgruppe entstehende Säure wird dann mit einer geeigneten Verbindung beispielsweise mit N-Hydroxysuccinimid, in den Rest R⁵ überführt. Hieraus wird die chemilumineszierende Acridiniumverbindung nach literaturbekannten Verfahren durch Alkylierung am Stickstoff in 10-Stellung gewonnen.

Die erhaltenen Acridiniumverbindungen lassen sich dann mit einer Substanz von biologischem Interesse, z.B. einem Antigen, einem Antikörper, einem Hormon, einem Arzneimittel, einem Arzneimittelmetaboliten, einem Toxin oder einem Alkaloid zu einer lumineszierenden Verbindung umsetzen. Dabei wird das Acridiniumderivat entweder direkt oder über ein Brückenmolekül wie Polylysin, Polyglutaminsäure oder Polyvinylamin an die biologisch interessante Substanz unter Ausbildung eines stabilen, immunologisch aktiven Konjugates gebunden. Dieses Konjugat wird auch als Tracer bezeichnet und wird in den nachfolgend beschriebenen Lumineszenzimmunoassays eingesetzt. Für den erfindungsgemäßen Lumineszenzimmunoassay zur Bestimmung einer antigenen Substanz in einer Flüssigkeitsprobe nach einem kompetitiven oder einem Sandwich-Verfahren wird mindestens eine immunologisch aktive Komponente benötigt, die auf einer festen Phase immobilisiert ist und außerdem der lumineszierende Tracer. Der Lumineszenzimmunoassay kann nun in verschiedener Weise durchgeführt werden.

Eine Möglichkeit besteht darin, daß man den mit dem Antigen spezifisch reagierenden immobilisierten Antikörper mit einer Probe der zu untersuchenden Flüssigkeit und einem Konjugat aus dem Antigen und einem chemilumineszierenden Acridiniumderivat (Antigen-Tracer) inkubiert, die Probe und den nicht gebundenen Tracer abtrennt, den gebundenen Tracer mit einem Oxidationsmittel zusammenbringt, um eine Lichtemission hervorzurufen, und dann aus der gemessenen Stärke der Lichtemission die Menge des vorhandenen Antigens bestimmt.

Eine andere Möglichkeit für die Durchführung des Lumineszenzimmunoassays besteht darin, daß man einen mit dem Antigen spezifisch reagierenden immobilisierten Antikörper mit einer Probe der zu untersuchenden Flüssigkeit mit einem Konjugat aus einem zweiten, spezifisch reagierenden Antikörper und einem chemilumineszierenden Acridinium-Derivat inkubiert, die Probe und das nicht gebundene, markierte Konjugat abtrennt, das gebundene, markierte Konjugat mit einem Oxidationsmittel zusammenbringt, um eine Lichtemission hervorzurufen, und aus der gemessenen Stärke der Lichtemission die Menge des vorhandenen Antigens bestimmt.

Die vorstehend genannten Lumineszenzimmunoassays können auch in der Weise durchgeführt werden, das man vor der Zugabe des markierten Konjugats die zu untersuchende Flüssigkeit vom immobilisierten Antikörper abtrennt.

In anderen erfindungsgemäß durchführbaren Lumineszenzimmunoassays ist nicht der Antikörper, sondern das Antigen immobilisiert. So kann ein mit dem Antikörper spezifisch reagierendes immobilisiertes Antigen mit einer Probe der zu untersuchenden Flüssigkeit und einer Lösung eines Konjugats aus dem Antikörper und einem chemilumineszierenden Acridiniumderivats inkubiert werden, dann die Probe und das nicht gebundene markierte Konjugat abgetrennt und dann das gebundene, markierte Konjugat mit einem Oxidationsmittel zusammengebracht werden. Dann tritt eine Lichtemission auf, aus deren Stärke die Menge des vorhandenen Antigens bestimmt werden kann.

Eine weitere Variante besteht darin, daß man ein mit dem Antikörper spezifisch reagierendes, immobilisiertes Antigen mit einer Lösung eines Konjugats aus dem Antikörper und einem chemilumineszierenden Acridiniumderivat inkubiert, das nicht umgesetzte, markierte Konjugat abtrennt, eine Probe der zu untersuchenden Flüssigkeit zufügt, die Probe anschließend wieder abtrennt, das gebundene, markierte Konjugat mit einem Oxidationsmittel zusammenbringt, um eine Lichtemission hervorzurufen und aus dieser dann die Menge des vorhandenen Antigens bestimmt.

Schließlich ist der Lumineszenzimmunoassay auch in der Weise durchführbar, daß man ein mit dem Antikörper spezifisch reagierendes, immobilisiertes Antigen mit einer Lösung eines Konjugats aus dem Antikörper und einem chemilumineszierenden Acridiniumderivat inkubiert, eine Probe der zu untersuchenden Flüssigkeit zufügt, die Probe und das nichtgebundene Konjugat abtrennt, das gebundene, markierte Konjugat mit einem Oxidationsmittel zusammenbringt und dann aus der gemessenen Lichtemission die Menge des vorhandenen Antigens bestimmt.

Die folgenden Beispiele - die bereits Gegenstand der Stammanmeldung sind - dienen als Leitfaden für die Herstellung und Verwendung der erfindungsgemäßen lumineszenten Verbindungen.

### Beispiel 1

### 9-Cyanacridin (1)

Zu Acridin (10 g) in 45 ml Ethanol gibt man 3,3 ml Eisessig und tropfenweise eine Lösung von 5,25 g Kaliumcyanid in 8 ml Wasser, erhitzt die Reaktionsmischung 2 Stunden am Rückfluß, kühlt ab und zieht die flüchtigen Bestandteile im Vakuum ab. Der Rückstand wird mit 30 ml 2 N NaOH verrührt, abgesaugt und nach zweimaligem Waschen mit 2 N NaOH und Wasser einige Zeit feucht an der Luft stehengelassen. Das Rohprodukt wird in Methylenchlorid verrührt, ungelöste Substanz wird abgesaugt, und mit Methylenchlorid gewaschen, die vereinigten organischen Phasen werden eingeengt und das rohe 9-Cyanacridin wird aus n-Butylacetat umkristallisiert.
Ausbeute: 50 % Schmelzpkt.: 183-5°C
IR: 2230 cm⁻¹

### Acridin-9-carbonsäure (2)

9-Cyanacridin (5 g) wird langsam portionsweise zu 40 ml konz. H₂SO₄ gegeben und die Mischung 2 Stunden auf 90-95°C erhitzt, nach Zugabe von 8,5 g NaNO₂ wird weitere 2 Stunden bei dieser Temperatur gerührt. Die heiße Lösung wird unter schnellem Rühren auf 620 ml Eiswasser gegeben, der Niederschlag abgesaugt und in möglichst wenig 2 N NaOH gelöst. Die Lösung wird filtriert und mit 50 %iger H₂SO₄ angesäuert, die ausfallende Acridin-9-carbonsäure abgesaugt und im Vakuum getrocknet.
Ausbeute: 95 % Schmelzpkt.: 288-9°C
IR: 3440(br), 3200(br), 2600-2500(br), 1980; 1650; 1605; 1420 cm⁻¹

### Acridin-9-carbonsäurechlorid-hydrochlorid (3)

Acridin-9-carbonsäure (5 g) wird portionsweise zu 50 ml frisch destilliertem SOCl₂ gegeben und 5 h am Rückfluß erhitzt; die dann klare Lösung wird durch Destillation bis zum beginnenden Niederschlag konzentriert, die Fällung wird durch Cyclohexanzugabe und Abkühlen vervollständigt. Absaugen des Niederschlages und Trocknung im Vakuum ergibt Acridin-9-carbonsäurechloridhydrochlorid.
Ausbeute: 90 % Schmelzpkt.: 223°C

| Elementaranalyse (berechnet als C₁₄H₉ClNO x HCl) | | | | |
|---|---|---|---|---|
| ber. | C 60,5 | H 2,8 | N 5,0 | Cl 25,5 |
| gef. | C 59,4 | H 3,3 | N 5,0 | Cl 25,2 |

### (Phenyl-2'-carbonsäure)acridin-9-thiocarboxylat (4)

Acridin-9-carbonsäurechlorid-hydrochlorid (30 g) wird in 720 ml Methylenchlorid suspendiert, Thiosalicylsäure (17,7 g) und 50 ml Triethylamin zugegeben, die klar werdende Lösung rührt man 10 Minuten bei Raumtemperatur nach. Nach Abziehen des Lösungsmittels wird der Rückstand mit 35 g Soda und 1400 ml Wasser versetzt, die resultierende Lösung bis zum Auftreten eines Niederschlags eingeengt, dieser wird abgesaugt. Das Filtrat wird mit NaCl gesättigt und der dabei ausfallende Niederschlag ebenfalls abgesaugt, Die wäßrige Lösung der vereinigten Niederschläge wird bei 80°C mit Eisessig angesäuert, das ausfallende Produkt abgesaugt und im Vakuum getrocknet.
Ausbeute: 80 % Schmelzpkt.: 261-5°C
NMR (DMSO, 100 MHz): δ= 7,6-8,4 ppm, komplexes Multiplett IR: 1680 cm⁻¹ (s), 1720 (m) 1260 (s)

### 2'-(Succinimidoyloxycarbonyl)phenylacridin-9-thiocarboxylat (5)

Zur Suspension von 10 g der Thiolestercarbonsäure im 190 ml trockenen Tetrahydrofuran gibt man bei 0°C 3,2 g N-Hydroxysuccinimid, dann bei -20°C 6,9 g Dicyclohexylcarbodiimid (DCC) zu und läßt bei -20°C 2 Stunden, dann über Nacht bei Raumtemperatur nachrühren. Nach Zugabe von 0,28 ml Eisessig wird 1 Stunde gerührt, dann Essigester (25 ml) zugegeben und vom Niederschlag abfiltriert. Das Filtrat wird eingeengt und ergibt nach Umkristallisation aus Chlorbenzol blaßgelbes
2'-(Succinimidoyloxycarbonyl)phenylacridin-9-thiocarboxylat.
Ausbeute: 80 % Schmelzpkt.: 198-200°C
IR: 1810 cm⁻¹, 1780, 1745, 1225, 1205
NMR (DMSO), 100 MHz : δ= 2,95 ppm (s, 4H), 7,7-8,4 ppm (m, 12H)

### 2'-(Succinimidoyloxycarbonyl)phenyl-10-methylacridinium-9-thiocarboxylat-tetrafluoroborat (6)

3 g N-Hydroxysuccinimidester (5) werden mit 7,8 g Trimethyloxoniumtetrafluoroborat in 40 ml 1,2-Dichlorethan 8 Stunden auf 80°C erhitzt und über Nacht bei Raumtemperatur nachgerührt. Der Niederschlag wird abfiltriert und mit 1,2-Dichlorethan ausgekocht. Die vereinigten organischen Phasen werden eingeengt und aus Aceton-Diisopropylether umkristallisiert.
Ausbeute: 40 % Schmelzpkt.: 245°C
IR: 3440 cm⁻¹ (br), 1800, 1780, 1740(s), 1670, 1065(s) NMR (DMSO, 100 MHz); δ= 3,0 ppm (s, 4H), 4,95 ppm (s, leicht verbreitert, 3H), 7,9-8,6 (m, 10H), 9,9 ppm (d, 2H)

### Beispiel 2

Die Darstellung von Succinimidoyloxycarbonylmethyl-10-methylacridinium-9-thiocarboxylat-tetrafluoroborat, ausgehend vom Säurechlorid (3) und Thioglykolsäure erfolgt analog der Synthese von (6). Die Ausbeuten der einzelnen Syntheseschritte sowie die spektroskopische Charakterisierung der Produkte (7) bis (9) sind nachfolgend angegeben:

### Carboxymethylacridin-9-thiocarboxylat (7)

Ausbeute: 60 % Schmelzpkt.: 218°C (unter Zersetzung) IR: 3440 cm⁻¹ (br), 2400(br), 1950(br), 1710(m), 1660(s), 1070(m)
NMR (DMSO, 100 MHz): δ= 4,25 ppm (s, 2H); 7,6-8,4 (m, 8H)

### Succinimidoyloxycarbonylmethylacridin-9-thiocarboxylat (8)

Ausbeute: 80 %
IR: 3440 cm⁻¹ (br), 2930, 1820, 1785, 1740(s), 1205, 1165 NMR (DMSO, 100 MHz); δ= 2,95 ppm (s, 4H), 4,77 ppm (s, 2H), 7,6-8,3 ppm (m, 8H)

### Succinimidoyloxycarbonylmethyl-10-methylacridinium-9-thiocarboxylat-tetrafluoroborat (9)

Ausbeute: 40 % Schmelzpkt.: 250°C
IR: 3440 cm⁻¹ (br), 1810, 1780, 1735(s), 1538, 1350, 1060 NMR (DMSO, 100 MHz): δ= 2,9 ppm (s, 4H), 4,8 (s, 2H), 4,9 ppm, (s, 3H), 7,7-9,0 (m, 8H)

### Beispiel 3

### N-Benzolsulfonyl-N-(benzyloxycarbonylmethyl)acridin-9-carbonsäureamid (10)

3,3 g N-Benzolsulfonylglycinbenzylester werden in 110 ml Tetrahydrofuran mit 130 mg 4-(Dimethylamino)-Pyridin und 6 ml Triethylamin versetzt, nach 10 Minuten gibt man 3 g Acridin-9-carbonsäurechlorid-hydrochlorid zu und erhitzt die entstehende Suspension 6 Stunden zum Rückfluß. Der Niederschlag wird abgesaugt, das Lösungsmittel abgezogen, der Rückstand in Methylenchlorid aufgenommen und kurz mit 2 N NaOH verrührt. Die organische Phase wird nach Trocknen über MgSO₄ eingeengt und der resultierende Rückstand aus Toluol/Heptan umkristallisiert.
Ausbeute: 70 % Schmelzpkt.: 58°C
IR: 3440 cm⁻¹ (br), 1735, 1680, 1357, 1165
NMR (DMSO, 100 MHz): δ= 5,2 ppm (s, 2H), 5,3 ppm (s, 2H), 7,0-8,4 ppm (m, 18H)

### N-Benzolsulfonyl-N-(carboxymethyl)acridin-9-carbonsäureamid (11)

1 g N-Benzolsulfonyl-N(benzyloxycarbonylmethyl)acridin-9-carbonsäureamid in 60 ml Eisessig wird unter Zusatz von 2 ml konzentrierter HCl und Pd/C (10 %) bei Raumtemperatur und Normaldruck hydriert; nach Beendigung der Reaktion wird der Katalysator abgesaugt, aus dem Filtrat erhält man bei Einengen die Carbonsäure als gelben Feststoff.
NMR (DMSO, 100 MHz): δ= 5,0 ppm (s, 2H), 7,1-8,5 ppm (m, 13H)

Die Verbindung (11) wird mit N-Hydroxysuccinimid analog zur Darstellung von (5) zu N-Benzolsulfonyl-N-(succinimidoyloxycarbonylmethylacridin-9-carbonsäureamid (12) umgesetzt. (12) wird mit Trimethyloxoniumtetrafluoroborat, wie bei (6) beschrieben, zu N-Benzolsulfonyl-N-(succinimidoyloxycarbonylmethyl)-10-methylacridinium-9-carbonsäureamidtetrafluoroborat (13) quaternisiert.

### Beispiel 4:

### N-Phenyl-N(4-benzyloxycarbonylbenzolsulfonyl)acridin-9-carbonsäureamid (14)

11g 4(N-Phenylsulfamido)benzoesäurebenzylester werden in 300 ml Methylenchlorid mit 360 mg 4-(Dimethylamino)pyridin und 16.6 ml Triethylamin versetzt, nach 10 Minuten gibt man 8.34 g Acridin-9-carbonsäurechlorid-hydrochlorid (3) zu und erhitzt 16 Stunden zum Rückfluß. Die abgekühlte Lösung wird kurz mit 2N NaOH verrührt, die abgetrennte organische Phase mit Wasser gewaschen, über Na₂SO₄ getrocknet und eingeengt. Der Rückstand wird aus Toluol/Heptan umkristallisiert.
Ausbeute: 70 % Schmelzpunkt 161 - 163°C NMR(DMSO, 100 MHz): δ= 5,5 ppm (s,2H), δ= 6.8-8.6 ppm (m,22H)

### N-Phenyl-N-(4-carboxybenzolsulfonyl)acridin-9-carbonsäureamidhydrobromid (15)

8,58 g N-Phenyl-N-(4-benzyloxycarbonylbenzolsulfonyl)acridin-9-carbonsäureamid (14) werden in 30 ml 33 % HBr in Eisessig 2 Stunden auf 60°C erhitzt, nach Abkühlen fügt man 60 ml Diisopropylether hinzu, saugt den Niederschlag ab und trocknet im Vakuum.
Ausbeute: 95 % Schmelzpunkt: 255°C NMR(DMSO, 100 MHz): δ= 6.8-9 ppm (m)

### N-Phenyl-N-(4-succinimidoyloxycarbonylbenzolsulfonyl)-acridin-9-carbonsäureamid (16)

5,63 g N-Phenyl-N-(4-carboxybenzolsulfonyl)acridin-9-carbonsäureamid-hydrobromid (15) in 250 ml Tetrahydrofuran werden mit 2,8 ml Triethylamin versetzt, auf -15°C abgekühlt und mit 0,96 ml Chlorameisensäureethylester versetzt. Man rührt 20 Minuten nach, gibt 1,15 g N-Hydroxysuccinimid zu, rührt 3 Stunden bei -15°C, läßt auf Raumtemperatur auftauen und über Nacht nachrühren. Vom Niederschlag wird abgesaugt, das Filtrat eingeengt, der Rückstand mit Methylenchlorid aufgenommen, die resultierende Lösung mit Wasser, NaHCO₃-Lösung und Wasser gewaschen und über Na₂SO₄ getrocknet. Die organische Phase wird eingeengt und der Rückstand aus Toluol umkristallisiert.
Ausbeute: 50 % Schmelzpunkt 226° (Zersetzung)
NMR(DMSO, 100 MHz): δ= 2,95 ppm (s,4H), δ= 6.8-8.7 ppm (m, 17H)

### N-Phenyl-N-(4-succinimidoyloxycarbonylbenzolsulfonyl)-10-methylacridinium-9-carbonsäureamid-fluorosulfonat (17)

1,16 g N-Phenyl-N-(4-succinimidoyloxycarbonylbenzolsulfonyl)-acridin-9-carbonsäureamid (16) werden in 60 ml 1,2-Dichlorethan mit 0.3 ml Methylfluorosulfonat 24 Stunden bei Raumtemperatur gerührt, der ausfallende Niederschlag wird abgesaugt und im Vakuum getrocknet.
Ausbeute: 65 %
IR: 3420 cm⁻¹ (br), 3100(br), 1805(w), 1770(m) 1745(s), 1700(m), 1385(m), 1280(m), 1255(s), 1230(s), 1205(s)
NMR(DMSO, 100 MHz): δ= 2,95 ppm (s,4H), δ= 4.75 ppm (s,br, 3H), δ= 7.0-9.0 ppm (m,17H)
Massenspektrum: m/z = 594 : M⁺ (Kation)

### Beispiel 5

Die Darstellung von N-(4-Methoxyphenyl)-N-(4-succinimidoyloxycarbonylbenzolsulfonyl)-10-methylacridinium-9-carbonsäureamid-fluorosulfonat (21) ausgehend von 4-[N-(4'-Methoxyphenyl)sulfamido]-benzoesäurebenzylester und Acridin-9-carbonsäurechlorid-hydrochlorid (3) erfolgt analog der Synthese von (17) (s. Beispiel 4). Die Ausbeuten der einzelnen Syntheseschritte sowie die spektroskopische Charakterisierung sind nachfolgend angegeben.

### N-(4-Methoxyphenyl)-N-(4-benzyloxycarbonyl-benzolsulfonyl)-acridin-9-carbonsäureamid (18)

Ausbeute: 70 % Schmelzpunkt: 182-183°C
NMR(DMSO, 100 MHz): δ= 3,5 ppm (s,3H), δ= 5,5 ppm (s,2H), δ= 6.35-6.63 ppm (d,br,2H), δ= 7.05-7.2 ppm (d,br,2H), δ= 7.35-8.5 ppm (m,17H)

### N - (4 -Methoxyphenyl ) -N - (4 -carboxybenzolsulfonyl )acridin -9-carbonsäureamid-hydrobromid (19)

Ausbeute: 95 % Schmelzpunkt: 273°C (Zersetzung)
NMR(DMSO, 100 MHz): δ= 3.5 ppm (s,3H), δ= 6.4-6.6 ppm (d,br, 2H), δ= 7.05-7.2 ppm (d,br,2H), δ= 7.7-8.5 ppm (m, 12H)

### N-(4-Methoxyphenyl)-N-(4-succinimidoyloxycarbonylbenzol= sulfonyl)acridin-9-carbonsäureamid (20)

Ausbeute: 50 % Schmelzpunkt: 232-234°C
NMR(DMSO,100 MHz): δ= 2,95 ppm (s,4H), δ= 3.5 ppm (s,3H), δ= 6.4-6.6 ppm (d,br,2H), δ=7.05-7.25 ppm (d,br,2H), δ= 7.8-8.6 ppm (m,12H) IR: 3050 cm⁻¹ 1805(w), 1780(m), 1740(s), 1700(m), 1505(m), 1370(m), 1250(m), 1200(s), 1185

### N-(4-Methoxyphenyl)-N-(4-succinimidoyloxycarbonylbenzol= sulfonyl)-10-methylacridinium-9-carbonsäureamid-fluoro= sulfonat (21)

Die Substanz fällt bei der Reaktion nicht aus, sie wird durch Einengen der Lösung und Verrühren des Rückstandes mit Diisopropylether gewonnen.
Ausbeute: 80 %
NMR(DMSO, 100 MHz): δ= 2,95 ppm (s,4H), δ= 3,5 ppm (s,3H), δ= 4.8 ppm (s,br,3H), δ= 6.45-6.7 ppm (d,br,2H), δ= 7.2-7.4 ppm (d,br,2H), δ= 7.7-9 ppm (m,12H)
Massenspektrum: m/z= 624 M⁺ (Kation)
IR: 3440 cm⁻¹ (br), 3100, 2950, 1805(w), 1775(m), 1740(s), 1695(m), 1610(m), 1505(m), 1375(m), 1280(m), 1250(s), 1205(s).

### Beispiel 6

Die Darstellung von N-(4-Methoxyphenyl)-N-(3-succinimidoyloxycarbonyl-benzolsulfonyl)-10-methylacridinium-9-carbonsäureamid-fluorosulfonat(25) ausgehend vom 3-[N-(4'-Methoxyphenyl)sulfamido]-benzoesäurebenzylester und Acridin-9-carbonsäurechlorid-hydrochlorid (3) erfolgt analog zur Synthes von (17) (s. Beispiel 4). Die Ausbeute der einzelnen Syntheseschritte sowie die spektroskopische Charakterisierung sind nachfolgend angegeben.

### N-(4-Methoxyphenyl)-N(3-benzyloxycarbonylbenzolsulfonyl)-acridin-9-carbonsäureamid (22)

Ausbeute: 70 % Schmelzpunkt 168-170°C
NMR(DMSO, 100 MHz): δ= 3.5 ppm (s,3H), δ=5.45 ppm (s,2H), δ= 6.5 ppm (s,br,2H), δ= 7.1 ppm (br,2H), δ= 7.3-8.8 ppm (m,17H)

### N-(4-Methoxyphenyl)-N-(3-carboxybenzolsulfonyl)acridin-9-carbonsäureamid-hydrobromid (23)

Ausbeute: 90 % Schmelzpunkt: 264°C NMR(DMSO, 100 MHz): δ= 3.5 ppm (s,3H), δ= 6.4-6.6 ppm (d,br,2H), δ= 7.0-7.2 ppm (d,br,2H), δ= 7.6-8.8 ppm (m,12H)

### N-(4-Methoxyphenyl)-N-(3-succinimidoyloxycarbonylbenzol= sulfonyl)acridin-9-carbonsäureamid (24)

Ausbeute: 50 % Schmelzpunkt: 223-225°C NMR (DMSO, 100 MHz): δ= 2.95 ppm (s,4H), δ= 3.5 ppm (s,3H), δ=6.4-6.6 ppm (d,br,2H), δ= 7.0-7.2 ppm (d,br,2H), δ= 7.4-8.9 ppm (m,12H)
IR: 3500 cm⁻¹ (br), 3060, 2950, 2840, 1805(w), 1785(m), 1740(s), 1700(m), 1510(m), 1380(m), 1250(m), 1205(m), 1165(m)

### N-(4-Methoxyphenyl)-N-(3-succinimidoyloxycarbonylbenzol= sulfonyl)-10-methylacridinium-9-carbonsäureamidfluorosulfonat (25)

Ausbeute: 90 %
NMR(DMSO, 100 MHz): δ= 2.95 ppm (s,4H), δ= 3.55 ppm (s,3H), δ= 4.8 ppm (s,br,3H), δ= 6.45-6.7 (d,br,2H), δ= 7.05-7.3 ppm (d,br,2H), δ= 7.5-8.9 ppm (m,12H)
IR: 3500 cm⁻¹ (br), 3080, 2950, 1805(w), 1780(m), 1740(s),35 1700(m), 1610(m), 1510(m), 1380(m), 1250(s), 1205(s), 1170(s)
Masse: m/z= 624 M⁺ (Kation)

### Beispiel 7

### Tracer-Herstellung für den α-Fetoprotein-Chemilumineszenzimmunoassay

100 µl Antikörper (1 mg/ml), 11,5 µl des nach Beispiel 1 hergestellten Acridiniumthioesters (Verbindung (6)) (1 mg/ml in DMSO und 600 µl Konjugationspuffer (0.01 M Phosphat, pH 8.0)) werden 15 Minuten inkubiert. Danach werden 200 µl Lysin (10 mg/ml) zugefügt und weitere 15 Minuten inkubiert. Dieser Ansatz wird auf eine PD 10-Säule (Sephadex G 25 Medium, 0.1 M Phosphat, pH 6.3 als Fließmittel) transferiert. 10 Tropfen/Fraktion werden gesammelt. Die einzelnen Fraktionen werden nach geeigneter Verdünnung auf ihre Chemilumineszenzaktivität getestet (350 µl Oxidationsmittel: 0.1 % H₂O₂ in 0.1 N NaOH). Die Tracerfraktionen (1. Aktivitätspeak) werden gepoolt und bei 4°C gelagert.

### Beispiel 8

### Durchführung des α-Fetoprotein Chemilumineszenzimmunoassays

50 µl Standard/Probe und 150 µl Puffer (Phosphat; 0.1 M pH 6.3, 1 % Tween 20, 0.1 % Rinderserumalbumin, 0.1 M NaCl, 0.01 % NaN₃) werden 15 Minuten in mit monoklonalem Anti-AFP-Antikörper beschichteten Röhrchen geschüttelt.
Danach wird 2 x mit 1 ml Puffer gewaschen.
200 µl Tracer werden in geeigneter Verdünnung zugeführt und 15 Minuten geschüttelt. Erneut wird 2 x mit 1 ml Puffer gewaschen. Die Chemilumeszenz-Messung wird mit 350 µl Oxidationsmittel (0.1 % H₂O₂ in 0.1 N NaOH, 2 sec. Meßzeit) gestartet.

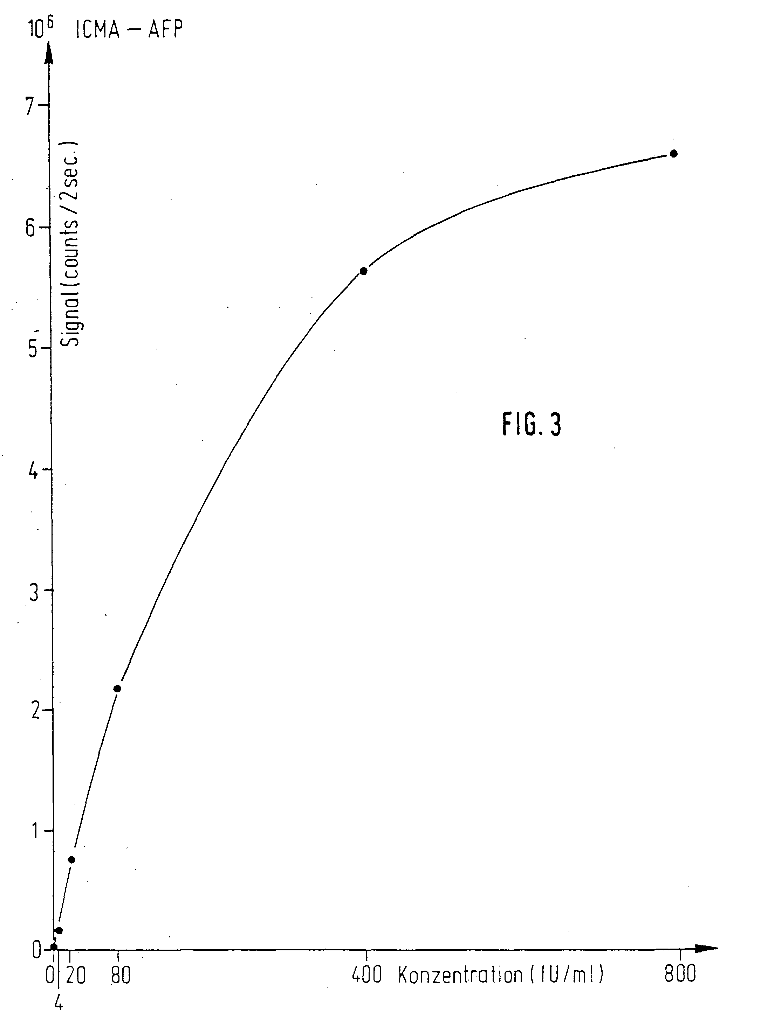

Figur 3 zeigt den typischen Verlauf einer Standardkurve eines Immuno-chemiluminometrischen Assays (ICMA) für das Alfa-Fetoprotein (AFP).

## Patentansprüche

1. Lumineszente Verbindung, **gekennzeichnet durch** ein Acridiniumderivat der Formel I 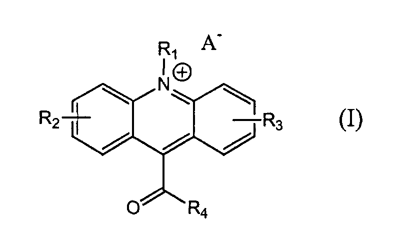 in der
R₁ Wasserstoff, ein Alkyl-, Alkenyl- oder Alkinylrest mit 1 bis 10 Kohlenstoffatomen, eine Benzyl-, Phenyl- oder Naphtylgruppe ist,
R₂ und R₃ Wasserstoff, eine Alkylgruppe mit 1 bis 4 Kohlenstoffatomen, eine Aminogruppe oder eine mono- oder di-C₁-C₄-Alkylaminogruppe ist, wobei die Alkylreste mit Hydroxy monosubstituiert sein können, sowie Morpholin, eine Carboxy-, C₁-C₄-Alkoxy-, Cyano-, Nitrogruppe oder Halogen sind,
R₄ einen Rest darstellt, in dem eine substituierte Sulfonamidgruppe über den Stickstoff direkt an die Carbonylgruppe gebunden ist und
A⁻ ein die Chemilumineszenz nicht beeinträchtigendes Anion ist,
ausgenommen sind Acridiniumverbindungen der Formel I, in denen
R₁, R₂ und R₃ wie oben definiert sind und
R₄ eine substituierte Sulfonamidgruppe der Formel V oder VI 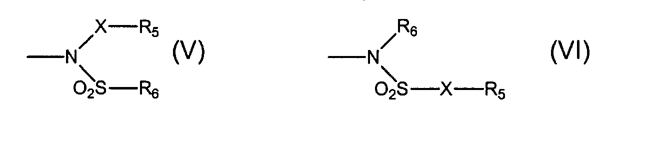 ist, wobei
X eine verzweigte oder unverzweigte C₁-C₅-Alkylengruppe oder eine Phenylen- oder Naphthylengruppe ist oder Chinol, Indol, Pyrrol oder Pyridin ist,
R₅ eine reaktive Gruppe ist, die unter schonenden Bedingungen selektiv mit Amino-, Carboxy-, Thiol- oder anderen funktionellen Gruppen in Substanzen von biologischem Interesse eine Bindung eingehen kann, und
R₆ in der Formel V ein Alkyl- oder Alkenylrest mit 1 bis 10 Kohlenstoffatomen, eine mit C₁-C₄ Alkyl mono- oder disubstituierte Aminogruppe, eine Morpholino-, eine Benzyl- oder eine Phenyl- oder Naphthylgruppe ist, die ggf. mit Hydroxy, Amino, Alkoxy mit 1 bis 4 Kohlenstorfatomen oder Phenoxy- oder Naphthoxygruppen substituiert ist, und
R₆ in der Formel VI zusätzlich zu den genannten Substituenten auch noch Wasserstoff bedeuten kann,
wobei das Acridiniumderivat der Formel I direkt oder über ein Brückenmolekül an ein Protein, ein Polypeptid oder an eine andere Substanz von biologischem Interesse unter Ausbildung eines stabilen, immunologisch aktiven Konjugates gebunden ist.

2. Verbindung nach Anspruch 1, wobei die Substanz von biologischem Interesse ausgewählt ist aus der Gruppe: Antigene, Hormone, Steroide, Arzneimittel, Arzneimittelmetabolite, Toxine, Alkaloide und Antikörper.

3. Lumineszenzimmunoassay zur Bestimmung einer antigenen Substanz in einer Flüssigkeitsprobe, bei dem in einem kompetitiven oder einem Sandwich-Verfahren mindestens eine immunologisch aktive Komponente auf einer festen Phase immobilisiert ist und mindestens eine weitere Komponente, der Tracer, eine lumineszente Verbindung entsprechend Anspruch 1 darstellt.

4. Lumineszenzimmunoassay nach Anspruch 3 **dadurch gekennzeichnet, daß** man
a) einen mit einem Antigen spezifisch reagierenden, immobilisierten Antikörper mit einer Probe der zu untersuchenden Flüssigkeit und einem Konjugat aus dem Antigen und einem chemilumineszierenden Acridiniumderivat der Formel I nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

5. Lumineszenzimmunoassay nach Anspruch 4 **dadurch gekennzeichnet, daß** vor der Zugabe des markierten Konjugates die zu untersuchende Flüssigkeit vom immobilisierten Antikörper abgetrennt wird.

6. Lumineszenzimmunoassay nach Anspruch 3 **dadurch gekennzeichnet, daß** man
a) einen mit einem Antigen spezifisch reagierenden, immobilisierten Antikörper mit einer Probe der zu untersuchenden Flüssigkeit und einem Konjugat aus einem zweiten, spezifisch reagierenden Antikörper und einem chemilumineszierenden Acridiniumderivat der Formel I nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

7. Lumineszenzimunoassay nach Anspruch 6 **dadurch gekennzeichnet, daß** vor der Zugabe des markierten Konjugats die zu untersuchende Flüssigkeit vom immobilisierten Antikörper abgetrennt wird.

8. Lumineszenzimmunoassay nach Anspruch 3 **dadurch gekennzeichnet, daß** man
a) ein mit einem Antikörper spezifisch reagierendes, immobilisiertes Antigen mit einer Probe der zu untersuchenden Flüssigkeit und einer Lösung des Konjugats aus dem Antikörper und einem chemilumineszierenden Acridiniumderivat der Formel I nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

9. Lumineszenzimmunoassay nach Anspruch 3 **dadurch gekennzeichnet, daß** man
a) ein mit einem Antikörper spezifisch reagierendes, immobilisiertes Antigen mit einer Lösung des Konjugats aus dem Antikörper und einem chemilumineszierenden Acridiniumderivat der Formel I nach Anspruch 1 inkubiert;
b) das nicht umgesetzte, markierte Konjugat abtrennt;
c) eine Probe der zu untersuchenden Flüssigkeit zufügt;
d) die Probe anschließend wieder abtrennt,
e) das gebundene, markierte Konjugat mit einem Oxidationsmittel zusammenbringt, um eine Lichtemission hervorzurufen und
f) aus der gemessenen Stärke der Lichtemission die Menge des vorhandenen Antigens bestimmt.

10. Lumineszenzimmunoassay nach Anspruch 3 **dadurch gekennzeichnet, daß** man
a) ein mit dem Antikörper spezifisch reagierendes, immobilisiertes Antigen mit einer Lösung des Konjugats aus dem Antikörper und einem chemilumineszierenden Acridiniumderivat der Formel I nach Anspruch 1 inkubiert;
b) eine Probe der zu untersuchenden Flüssigkeit zufügt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

## Claims

1. A luminescent compound which comprises an acridinium derivative of the formula I 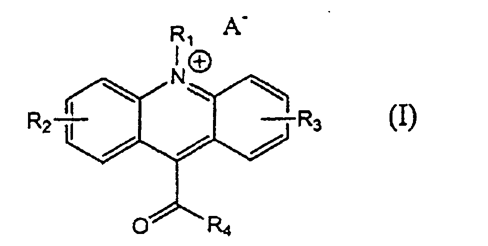 in which
R₁ is hydrogen, an alkyl, alkenyl or alkynyl radical with 1 to 10 carbon atoms or a benzyl, phenyl or naphthyl group,
R₂ and R₃ are hydrogen, an alkyl group with 1 to 4 carbon atoms, an amino group or a mono- or di-C₁-C₄ alkylamino group, where the alkyl radicals can be monosubstituted by hydroxyl, and morpholine, a carboxyl, C₁-C₄-alkoxy, cyano or nitro group or halogen,
R₄ is a radical in which a substituted sulfonamide group is bonded via the nitrogen directly to the carbonyl group, and
A⁻ is an anion which does not impair the chemiluminescence,
excluding acridinium compounds of the formula I in which R₁, R₂ and R₃ are each as defined above and R₄ is a substituted sulfonamide group of the formula V or VI 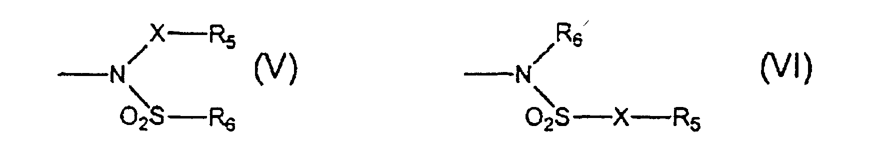 where
X is a branched or unbranched C₁-C₅-alkylene group or is a phenylene or naphthylene group or is quinole, indole, pyrrole or pyridine,
R₅ is a reactive group which under mild conditions is capable of bonding selectively with amino, carboxyl, thiol or other functional groups in substances of biological interest, and
R₆ in the formula V is an alkyl or alkenyl radical having 1 to 10 carbon atoms, a C₁-C₄-alkyl-mono-substituted or -disubstituted amino group or is a morpholino, benzyl, phenyl or naphthyl group which is optionally substituted by hydroxyl, amino, alkoxy of 1 to 4 carbon atoms or phenoxy or naphthoxy groups, and
R₆ in the formula VI, as well as the substituents mentioned, may additionally be hydrogen,
where the acridinium derivative of the formula I is bonded directly or via a bridge molecule to a protein, a polypeptide or another substance of biological interest to form a stable immunologically active conjugate.

2. A compound as claimed in claim 1, wherein the substance of biological interest is selected from the group consisting of antigens, hormones, steroids, drugs, drug metabolites, toxins, alkaloids and antibodies.

3. A luminescence immunoassay for the determination of an antigenic substance in a liquid sample, in which, in a competitive process or a sandwich process, at least one immunologically active component is immobilized on a solid phase and at least one other component, the tracer, is a luminescent compound as claimed in claim 1.

4. A luminescence immunoassay as claimed in claim 3, which comprises
a) incubating an immobilized antibody, which reacts specifically with an antigen, with a sample of the liquid under investigation and a conjugate of the antigen and a chemiluminescent acridinium derivative of the formula I as claimed in claim 1;
b) separating off the sample and the unbound labeled conjugate;
c) bringing the bound labeled conjugate together with an oxidizing agent in order to cause photoemission and
d) determining the amount of antigen present from the photoemission intensity measured.

5. A luminescence immunoassay as claimed in claim 4, wherein the liquid under investigation is separated off from the immobilized antibody before addition of the labeled conjugate.

6. A luminescence immunoassay as claimed in claim 3, which comprises
a) incubating an immobilized antibody, which reacts specifically with an antigen, with a sample of the liquid under investigation and a conjugate of a second, specifically reacting antibody and a chemiluminescent acridinium derivative of the formula I as claimed in claim 1;
b) separating off the sample and the unbound labeled conjugate;
c) bringing the bound labeled conjugate together with an oxidizing agent in order to cause photoemission and
d) determining the amount of antigen present from the photoemission intensity measured.

7. A luminescence immunoassay as claimed in claim 6, wherein the liquid under investigation is separated off from the immobilized antibody before addition of the labeled conjugate.

8. A luminescence immunoassay as claimed in claim 3, which comprises
a) incubating an immobilized antigen, which reacts specifically with an antibody, with a sample of the liquid under investigation and a solution of the conjugate of the antibody and a chemiluminescent acridinium derivative of the formula I as claimed in claim 1;
b) separating off the sample and the unbound labeled conjugate;
c) bringing the bound labeled conjugate together with an oxidizing agent in order to cause photoemission and
d) determining the amount of antigen present from the photoemission intensity measured.

9. A luminescence immunoassay as claimed in claim 3, which comprises
a) incubating an immobilized antigen which reacts specifically with an antibody, with a solution of the conjugate of the antibody and a chemiluminescent acridinium derivative of the formula I as claimed in claim 1;
b) separating off the unreacted labeled conjugate;
c) adding a sample of the liquid under investigation;
d) subsequently separating off the sample again;
e) bringing the bound labeled conjugate together with an oxidizing agent in order to cause photoemission and
f) determining the amount of antigen present from the photoemission intensity measured.

10. A luminescence immunoassay as claimed in claim 3, which comprises
a) incubating an immobilized antigen, which reacts specifically with the antibody, with a solution of the conjugate of the antibody and a chemiluminescent acridinium derivative of the formula I as claimed in claim 1;
b) adding a sample of the liquid under investigation;
c) bringing the bound labeled conjugate together with an oxidizing agent in order to cause photoemission and
d) determining the amount of antigen present from the photoemission intensity measured.

## Revendications

1. Composé luminescent, **caractérisé par** un dérivé d'acridinim 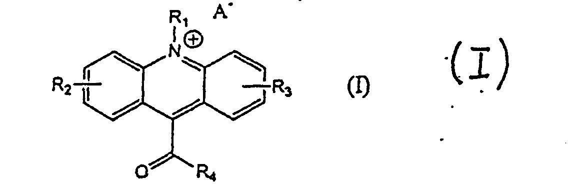 dans laquelle
R₁ est un atome d'hydrogène, un radical alkyle, alcényle ou alcynyle ayant de 1 à 10 atomes de carbone, un groupe benzyle, phényle ou naphtyle,
R₂ et R₃ représentent un atome d'hydrogène, un groupe allyle ayant de 1 à 4 atomes de carbone, un groupe amino ou un groupe mono- ou dialkyl(C₁-C₄)amino, les radicaux alkyle pouvant être monosubstitués par le groupe hydroxy, ainsi que le radical morpholine, un groupe carboxy, alcoxy en C₁-C₄, cyano, nitro, ou un atome d'halogène,
R₄ représente un radical dans lequel un groupe sulfonamido substitué est lié directement par l'atome d'azote au groupe carbonyle, et
A⁻ est un anion ne nuisant pas à la chimiluminescence,
à l'exclusion des composés d'acridinium de formule I dans lesquels
R₁, R₂ et R₃ sont tels que définis plus haut et
R₄ est un groupe sulfonamido substitué de formule V ou VI 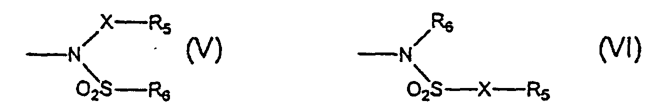
X étant un groupe alkylène en C₁-C₅ ramifié ou non ramifié, ou un groupe phénylène ou naphtylène, ou le radical quinolin, indole, pyrrole ou pyridine,
R₅ est un groupe réactif qui, dans des conditions ménagées, peut former-sélectivement une liaison avec des groupes amino, carboxy, thiol ou d'autres groupes fonctionnels dans des substances d'intérêt biologique et
R₆ dans la formule V représente un radical alkyle ou alcényle ayant de 1 à 10 atomes de carbone, un groupe amino mono- ou disubsitué par un ou des groupes alkyle en C₁-C₄, un groupe morpholino, un groupe benzyle, phényle ou naphtyle, qui est éventuellement substitué par des groupes hydroxy, amino, alcoxy ayant de 1 à 4 atomes de carbone ou phénoxy ou naphtoxy, et
R₆ dans la formule VI peut également représenter, en plus des substituants cités, encore un atome d'hydrogène,
le dérivé d'acridinium de formule I étant lié, directement ou par l'intermédiaire d'une molécule formant pont, à une protéine, un polypeptide ou à une autre substance d'intérêt biologique, avec formation d'un conjugué stable, à activité immunologique.

2. Composé selon la revendication 1, la substance d'intérêt biologique étant choisie dans le groupe constitué par les antigènes, hormones, stéroïdes, médicaments, métabolites de médicaments, toxines, alcaloïdes et anticorps.

3. Dosage immunologique par luminescence pour la détermination d'une substance antigénique dans un échantillon de liquide, dans lequel, dans un procédé compétitif ou en sandwich, au moins un composant immunologiquement actif est immobilisé sur une phase solide et au moins un autre composant, le marqueur, représente un composé luminescent selon la revendication 1.

4. Dosage immunologique par luminescence selon la revendication 3, **caractérisé en ce que**
a) on met un anticorps immobilisé, réagissant spécifiquement avec un antigène, à incuber avec un échantillon du liquide à analyser et un conjugué de l'antigène et d'un dérivé d'acridinium chimiluminescent de formule I selon la revendication 1 ;
b) on sépare l'échantillon et le conjugué marqué non lié ;
c) on met le conjugué marqué lié en contact avec un oxydant, afin de provoquer une émission de lumière ; et
d) on détermine la quantité de l'antigène présent à partir de l'intensité mesurée de l'émission de lumière.

5. Dosage immunologique par luminescence selon la revendication 4, **caractérisé en ce qu'**avant l'addition du conjugué marqué, on sépare les liquide à analyser de l'anticorps immobilisé.

6. Dosage immunologique par luminescence selon la revendication 3, **caractérisé en ce que**
a) on met un anticorps immobilisé, réagissant spécifiquement avec un antigène, à incuber avec un échantillon du liquide à analyser et un conjugué d'un second anticorps réagissant spécifiquement et d'un dérivé d'acridinium chimiluminescent de formule I selon la revendication 1 ;
b) on sépare l'échantillon et le conjugué marqué non lié ;
c) on met le conjugué marqué lié en contact avec un oxydant, afin de provoquer une émission de lumière ; et
d) on détermine la quantité de l'antigène présent à partir de l'intensité mesurée de l'émission de lumière.

7. Dosage immunologique par luminescence selon la revendication 6, **caractérisé en ce qu'**avant l'addition du conjugué marqué, on sépare le liquide à analyser de l'anticorps immobilisé.

8. Dosage immunologique par luminescence selon la revendication 3, **caractérisé en ce que**
a) on met un antigène immobilisé, réagissant spécifiquement avec un anticorps, à incuber avec un échantillon du liquide à analyser et une solution du conjugué de l'anticorps et d'un dérivé d'acridinium chimiluminescent de formule I selon la revendication 1 ;
b) on sépare l'échantillon et le conjugué marqué non lié ;
c) on met le conjugué marqué lié en contact avec un oxydant, afin de provoquer une émission de lumière ; et
d) on détermine la quantité de l'antigène présent à partir de l'intensité mesurée de l'émission de lumière,

9. Dosage immunologique par luminescence selon la revendication 3, **caractérisé en ce que**
a) on met un antigène immobilisé, réagissant spécifiquement avec un anticorps, à incuber avec une solution du conjugué de l'anticorps et d'un dérivé d'acridinium chimiluminescent de formule I selon la revendication 1 ;
b) on sépare le conjugué marqué n'ayant pas réagi ;
c) on ajoute un échantillon du liquide à analyser ;
d) on sépare ensuite de nouveau l'échantillon ;
e) on met le conjugué marqué lié en contact avec un oxydant, afin de provoquer une émission de lumière ; et
f) on détermine la quantité de l'antigène présent à partir de l'intensité mesurée de l'émission de lumière.

10. Dosage immunologique par luminescence selon la revendication 3, **caractérisé en ce que**
a) on met un antigène immobilisé, réagissant spécifiquement avec un anticorps, à incuber avec une solution du conjugué de l'anticorps et d'un dérivé d'acridinium chimiluminescent de formule I selon la revendication 1 ;
b) on ajoute un échantillon du liquide à analyser ;
c) on met le conjugué marqué lié en contact avec un oxydant, afin de provoquer une émission de lumière; et
d) on détermine la quantité de l'antigène présent à partir de l'intensité mesurée de l'émission de lumière.
